# EUROPÄISCHE PATENTANMELDUNG

(11) **EP 2 695 946 A1**
(43) Veröffentlichungstag der Anmeldung: **12.02.2014**
(21) Anmeldenummer: 12179820.1
(22) Anmeldetag: 09.08.2012
(51) Int. Cl.: C12P 5/02, B01D 53/00, C07C 43/04, C01B 3/36, C01B 3/38

(54) **Verfahren und Anlage zur Herstellung von Dimethylether**

(71) Anmelder: Methapower Biogas GmbH, 1010 Wien (AT)
(72) Erfinder: Gruber-Schmidt, Johann, 1180 Wien (AT)
(74) Vertreter: Sonn & Partner Patentanwälte

(57) **Zusammenfassung**

Die Erfindung betrifft ein Verfahren zur Herstellung von Dimethylether, umfassend die Schritte: Fermentierung von Biomasse zur Erzeugung von Rohbiogas enthaltend Methan und Kohlendioxid, Reinigung des Rohbiogases durch Abtrennung von Kohlendioxid, wodurch ein Methan angereichertes Rohbiogas erhalten wird, Herstellung von Synthesegas umfassend Kohlenmonoxid und Wasserstoff durch Umsetzung von Methan mit Sauerstoff, Herstellung von Dimethylether aus dem Synthesegas durch katalytische Kondensation des Synthesegases oder von dem Synthesegas als Zwischenschritt kondensiertem Methanol; sowie eine Produktionsanlage zur Herstellung von Dimethylether aus den Abgasen der Fermentierung von Biomasse.

## Beschreibung

Die vorliegende Erfindung betrifft kombinierte Reaktoren bei der Biogasaufbereitung und -verwertung.

Biogas beispielsweise ist ein Gemisch hauptsächlich aus Methan und Kohlendioxid (auch Kohlenstoffdioxid), üblicherweise im Bereich 45% bis 70% Methan und 30% bis 55% Kohlendioxid. Methan, aus Biogas oder aus Erdgas, wird einerseits zur Energieerzeugung oder als Brennstoff genutzt und ist ein industriell wertvoller Rohstoff. Die Weiterverarbeitung von Methan als chemisch synthetischer Ausgangsstoff erfolgt zumeist über die Stufe der Produktion von Synthesegas (auch "Syngas" bezeichnet).

Die Veröffentlichungen WO 2009/021710, DE 198 27 154 A1, GB2375353, US 7,687,250 und die DE 31 30 013 A1 beschreiben beispielsweise die Produktion von Synthesegas aus Biogas. Der WO 2009/021710 liegt die Aufgabe zugrunde, die Ausbeute bei der Herstellung von Synthesegas zu optimieren. In der DE 31 30 013 A1 wird das Synthesgas unter Verwendung von Hydrierungskatalysatoren in flüssige Kohlenwasserstoffe durch die Fischer-Tropsch-Synthese weiter umgewandelt. Die Veröffentlichungen DE 198 27 154 A1 und GB 2375353 betreffen die Umwandlung von Biogas mit Kohlendioxid über Synthesegas zu Methanol durch die Heat-pipe-Methanol-Synthese. Die US 7,687,250 beschreibt die Steuerung der Synthesegasinhaltsstoffe (Kohlenmonoxid und Wasserstoff) durch Justierung der Wasserdampf- und Methanverhältnisse und weiters die Produktion von Kohlenwasserstoffen aus Synthesegas durch die Fischer-Tropsch-Synthese.

In der WO 2005/118826 A1 wird die Produktion von Ethanol aus Synthesegas, welches aus der Fermentation gewonnen wurde, beschrieben.

Die WO 2010/149339 A1 beschreibt die Herstellung von Kohlenwasserstoffen aus Synthesegas, wobei das Synthesegas in einem ersten Schritt in Methanol, Dimethylether und höhere Alkohole umgewandelt wird, welche weiter zu den Kohlenwasserstoffen prozessiert werden. Synthesegas wird hierbei aus biogenen Stoffen wie Kohle und Koks gewonnen.

In der Veröffentlichung WO 2010/072424 A1 wird die Herstellung eines Synthesegases aus einem Erdölprodukt beschrieben, welches in weiteren Schritten, z.B. über eine Shift-Reaktion und Oxidation, in mehreren Schritten zu einem Methanol-/Dimethylethergemisch weiterverarbeitet wird.

Die Umwandlung von erhaltenem Synthesegas zu Dimethylether als Einschrittprozess - oder alternativ über die Synthese von Methanol als Zweischrittprozess - ist in der WO 2006/020083 A1 beschrieben. Als Ausgangsstoff wird Erdgas verwendet. Der Zweischrittprozess wird auch in der WO 96/23755 A1 beschrieben.

Eine verbesserte Dimethylethersynthese aus Synthesegas durch zusätzliche Waschschritte zur Kohlendioxid-Entfernung aus dem Synthesegas wird in der EP 2028173 B1 beschrieben.

In zahlreichen industriellen und landwirtschaftlichen Verfahren, beispielsweise in Kommunalbetrieben, bei der Verarbeitung von Nahrungsmitteln und Tierfutter und in der Forstwirtschaft wird Biomasse in der Form von Abfall- und Nebenprodukten erzeugt. Die landwirtschaftliche und chemische Industrie sowie öffentliche Betriebe haben ein erhebliches Interesse an der Entwicklung von Verfahren zur Umwandlung dieser Biomassen in Materialien mit einem höheren Nutzwert. So könnten derartige Biomassen beispielsweise unter Verwendung von Mikroorganismen und/oder hydrolytischen Enzymen potentiell in Bioethanol, Biogas oder Chemikalien umgewandelt werden. Allerdings haben die meisten der heutzutage bekannten Verfahren aufgrund ihrer hohen Produktionskosten und der hohen Energieerfordernisse und der infolgedessen innewohnenden unsicheren ökonomischen Realisierbarkeit noch nicht die kommerzielle Anwendung im Großmaßstab erreicht. Es ist daher ein Ziel vieler technologischer Entwicklungen Effizienzsteigerungen zu ermöglichen. Wird Rohbiogas aufbereitet bzw. aufgereinigt, damit es in Form von Biomethan vorliegt, kann dieses Biomethan in vor Ort lokalisierten Tankstellenanlagen direkt zur Verwendung als Kraftstoff gelagert werden. Die Speicherung größerer Mengen eines Gases ist jedoch kostspielig und eine bedarfsorientierte Produktion wäre wünschenswert.

Der Fermentierungsprozess ist ein langsamer und kontinuierlicher Prozess, der am besten in einen stabilen Volllastprozess gefahren und betrieben werden kann. Diese Prozessweise ermöglicht so die höchste und effizienteste Ausbeute an Rohbiogas. Aus regelungstechnischer Sicht bedeutet dies, dass es sich um einen sehr langsamen Regelprozess handelt, also ein schnelles Anfahren und Abfahren der Fermentierungsanlagen nur innerhalb von Tagen und Wochen erfolgen kann, abhängig von der Größenordung der Anlage und der verwendeten Rohstoffe (Biomasse).

Bei den klassischen Rohstoffen für die Nutzung in den Fermentern der Biogasanlagen zur Nutzung in der Umformung zu Biogas sind Mais, Hirse, also Körner, bekannt und Stand der Technik. Um nun weitere Stoffe für die Fermentierung nutzbar zu machen, muss ein Aufschluss der Stoffe so weit erfolgen, dass diese von den Mikroorganismen in den Fermentern der Biogasanlage umgewandelt werden können. Um Biomasse, neben Mais und Hirse, wie Pferdemist, Hühnermist, Kuhmist, Maisstroh, Getreidestroh, Gras, Klee, Luzerne sowie Bioabfälle für die Anwendung in Biogasanlagen verwendbar zu machen, bedingt also, dass diese Rohstoffe (Substrate) aufgeschlossen werden. Der Aufschluss der Biomasse bedeutet, dass diese so zerkleinert wird, dass ein verbesserter Aufschluss im Biogasreaktor (Fermenter) möglich ist. Dieser mechanische Aufschluss ist notwendig, um auch Biomassestoffe im Biogasfermenter nutzbar zu machen, die im Fermenter kaum oder nur sehr schwer zu Biogas verwertet werden können. Dieser Aufschluss ist eine wesentliche Grundlage der Erzeugung von Rohbiogas aus Reststoffen auf kostengünstiger Basis. Auch dieser Aufschlussprozess ist wie der Fermentationsprozess kein schneller Prozess, sondern als kontinuierlicher Prozess an den Fermentierungsprozess gekoppelt. Ein optimierter Aufschluss bedingt somit eine hohe Ausbeute an Rohbiogas aus Reststoffen, welche für den ohnehin niedrigen Wirkungsgrad von Biogasanlagen unabdingbar ist.

Derzeit ist die Produktion von biogenem Methan der Verwertung von Erdgas aufgrund aufwändiger Produktionsmethoden auf der Kostenseite unterlegen. Zudem ist bei der bedarfsorientierten Produktion von Methan mit hohen Nachfrageschwankungen zu rechnen, welche großen Aufwand zur Lagerung von Biomethan bedingt. Wünschenswert sind daher andere, leichter lagerbare Produkte von hohem Wert, welche flexibel in einer Biogasanlage produziert werden können und ebenfalls ohne aufwendige Transportkosten vor Ort genutzt werden können.

Diese Problematiken der Biogasfermentierung wird durch die vorliegende Erfindung gelöst. In einem ersten Aspekt betrifft die vorliegende Erfindung ein Verfahren zur Herstellung von Dimethylether umfassend die Schritte der
a) Fermentierung von Biomasse zur Erzeugung von Rohbiogas enthaltend Methan und Kohlendioxid,
b) Reinigung des Rohbiogases durch Abtrennung von Kohlendioxid, wodurch ein mit Methan angereichertes Rohbiogas erhalten wird,
c) Herstellung von Synthesegas umfassend Kohlenmonoxid und Wasserstoff durch Umsetzung von Methan mit Sauerstoff,
d) Herstellung von Dimethylether aus dem Synthesegas durch katalytische Kondensation des Synthesegases oder von dem Synthesegas als Zwischenschritt kondensiertem Methanol.

In einem weiteren Aspekt betrifft die Erfindung eine Produktionsanlage geeignet zur Herstellung von Dimethylether mit einem Fermenter, einem Rohbiogasreiniger zur Trennung von Methan und Kohlendioxid, mit einem Synthesegasreaktor und einem Dimethylether-Reaktor zur katalytischen Kondensation von Synthesegas zu Dimethylether. Diese Anlage kann zur Herstellung von Dimethylether verwendet werden, insbesondere gemäß dem erfindungsgemäßen Verfahren.

Die weitere Beschreibung der vorzugsweisen Ausführungsformen der vorliegenden Erfindung betrifft sowohl das erfindungsgemäße Verfahren als auch die Produktionsanlage, welche Mittel für einzelne Verfahrensschritte und Anlagenteile zur Durchführung enthalten kann bzw. wobei die beschriebenen Anlagenteile im erfindungsgemäßen Verfahren funktionsentsprechend eingesetzt werden können oder die in den Anlagenteilen ablaufenden Prozesse Schritte im erfindungsgemäßen Verfahren sein können. Insbesondere wird die vorliegende Erfindung weiters in den Ansprüchen definiert.

Der Begriff "Biomasse" wird im Stand der Technik unterschiedlich verwendet und betrifft auch nicht fermentierte Produkte wie Holz. Die Verwendung von fester Biomasse, wie z.B. Holz, als Grundlage für die Erzeugung von Methanol ist bekannt. Dazu gibt es verschiedene Verfahren zur Verflüssigung von fester Biomasse, wie die Erzeugung von Schwarzlaugen, Braunlaugen oder Verölungsverfahren. Schwarzlaugen und Braunlaugen sind Produkte aus Kochprozessen in der Zellstoff und Holzindustrie. Die vorliegende Erfindung hingegen bezieht sich auf Biomasse, welche fermentiert wird, d.h. durch Mikroorganismen aufgeschlossen wird. Wie einleitend erwähnt, ist die Fermentation ein langsamer Prozess, der schlecht reguliert werden kann. Insbesondere sind mit dem Prozessstart aber mit dem Auslauf hoher Aufwand bzw. hohe Energieverluste verbunden, welche die ohnehin niedrigen Wirkungsgrade stark beeinträchtigen. Daher war es insbesondere notwendig, für die Fermentierung Bedingungen zu schaffen, die einen kontinuierlichen Betrieb ermöglichen. Da jedoch in den seltensten Fällen, insbesondere nicht bei Kleinanlagen, eine kontinuierliche und konstante Produktabnahme möglich ist, müssten neue Verwertungsmöglichkeiten geschaffen werden, die speziell vor Ort benötigt werden. Daher betrifft die Erfindung die Herstellung eines in der Landwirtschaft brauchbaren Produkts, Dimethylether (DME), als Alternative zu Biogas (Methan).

Dimethylether kann als alternativer Kraftstoff für umfunktionierte Dieselfahrzeuge wie Traktoren, Zugmaschinen, Holzerntemaschinen (Harvester, Skidder, Fällerbündler), aber auch bei Zug- und Transportfahrzeugen in der Holzindustrie, Bauwirtschaft und Spedition verwendet werden. Der Umbau eines Dieselmotors zur Nutzung von DME als Kraftstoff ist bekannt (JP 2003/097307, CN 1556319). Für derartige Anwendungen ist die erfindungsgemäße Produktionsanlage mit einer Tankstelle im Vertrieb von DME verbunden. Vorzugsweise ist die erfindungsgemäße Produktionsanlage mit einem landwirtschaftlichen Betrieb verbunden, welcher sowohl die Biomasse zur Verfügung stellt als auch die Abnahme für DME gewährleistet.

Üblicherweise wird gemäß der Erfindung bei der Fermentierung der Biomasse ein Rohbiogas umfassend 40% bis 65% Methan und 35% bis 55% Kohlendioxid erhalten. Alle %-Angaben von Gasgemischen beziehen sich auf vol.-%. Dieser Schritt kann in einem Fermenter durchgeführt werden.

Biogas entsteht durch den natürlichen Prozess des mikrobiellen Abbaus organischer Stoffe unter anoxischen Bedingungen. Dabei setzen Mikroorganismen die enthaltenen Kohlenhydrate, Eiweiße und Fette in die Hauptprodukte Methan und Kohlenstoffdioxid um. Verfahren zur Fermentierung zur Bildung von Biogas sind unter anderem in der WO 03/06387 A2, EP 0646756 A1, WO 2009/137948 A2, DE 3243103 A1, beschrieben und können erfindungsgemäß zur Fermentierung der Biomasse eingesetzt werden.

Der biologische Prozess besteht aus mehreren Stufen, die jeweils von Mikroorganismen verschiedener Stoffwechseltypen durchgeführt werden. Polymere Bestandteile der Biomasse, wie Zellulose, Hemizellulose, Proteine, werden zunächst durch mikrobielle Exoenzyme zu monomeren (niedermolekularen) Stoffen umgewandelt. Niedermolekulare Stoffe werden durch gärende Mikroorganismen zu Alkoholen, organischen Säuren, Kohlenstoffdioxid (CO₂) und Wasserstoff (H₂) abgebaut. Die Alkohole und organischen Säuren werden durch acetogene Bakterien zu Essigsäure und Wasserstoff umgesetzt. In der letzten Stufe werden durch methanogene Archaeen aus Kohlenstoffdioxid, Wasserstoff und Essigsäure die Endprodukte Methan (CH₄) und Wasser gebildet. Geeignete methanogene Bakterienstämme, welche Methan bei der Fermentierung bilden, sind beispielsweise in der DE 3243103 A1 genannt. Solche sind z.B. Mikroorganismen der Gattungen Methanobacterium, Methanobacillus, Methanosarcina oder Methanospirillum. Solche können der Biomasse zur Fermentierung zugesetzt werden. Erwärmte Biomasse wird üblicherweise einem Fermenter zugeleitet, worin bei 36°C-40 °C Biogas gebildet wird. Je nach Biomassezusammensetzung kann Wasser, Bakterienkulturen und Startermedium zugesetzt werden, um die Fermentation in Gang zu setzen.

Vor der Biogasaufbereitung besteht die wassergesättigte Gasmischung aus den Hauptkomponenten Methan (CH₄) und Kohlenstoffdioxid (CO₂). In Spuren sind meist auch Stickstoff (N₂), Sauerstoff (O₂), Schwefelwasserstoff (H₂S), Wasserstoff (H₂) und Ammoniak (NH₃) enthalten. Für die Verwertung von Biogas ist der Methananteil am wichtigsten, da seine Verbrennung Energie freisetzt. Die Bezeichnung Biogas wird zusammenfassend für energiereiche Gase verwendet, die unter anoxischen Bedingungen aus organischen Stoffen entstehen.

Die Fermentierung umfasst im Speziellen den mikrobiellen Abbau organischer Stoffe unter anoxischen Bedingungen. Die eingesetzte Biomasse als organische Stoffe enthält vorzugsweise Pflanzenmaterial oder Viehmaterial, insbesondere Zellulose, Lignine, Proteine. Die Biomasse wird durch herkömmliche Mikroorganismen der Fermentierung unter Freisetzung von unter anderem Methan und CO₂ umgesetzt.

Vorzugsweise umfasst die Fermentierung den Abbau von organischen Stoffen ausgewählt aus Pflanzensilage, insbesondere Maissilage oder Grassilage, Stroh, insbesondere Maisstroh oder Getreidestroh, Gras, Klee, Luzerne, Viehmist oder -dung, insbesondere Schweine-, Hühner-, Kuh-, oder Pferdemist oder Gülle, Bioabfall oder -müll, Haushalts-Biomüll, Pflanzenschlempe, Maische oder Kompost, Industrieabfall der Getränkeindustrie und Lebensmittelindustrie, Brotreste, verdorbene Lebensmittel. Die Verwertung von energiereichen Pflanzen (d.h. Pflanzen mit hoher Methanausbeute) oder Pflanzenteilen ist ebenfalls möglich. Derartige Pflanzen sind beispielsweise Mais, insbesondere Maiskolben oder Hirse. Die vorliegende Erfindung entfaltet ihre Vorteile insbesondere bei der Verwertung von Abfallprodukten. Da derartige energiereiche Pflanzen jedoch auch anderweitige Verwendungsmöglichkeiten besitzen (z.B. als Nahrungsmittel), werden sie vorzugsweise nicht erfindungsgemäß eingesetzt. In besonders bevorzugten Ausführungsformen der vorliegenden Erfindung werden daher keine menschlichen Nahrungsmittel eingesetzt. Dies ist für den erfindungsgemäßen Prozess nicht abträglich, da durch den optimierten Prozess und der verbesserten Produktverwertung auch niedrigenergene Abfallprodukte wirtschaftlich verwertet werden können.

Vorzugsweise umfasst die Fermentierung zusätzlich die Nutzung eines Nachgärprozesses, um aus dem Gärrest noch weiteres Rohbiogas zu gewinnen.

Die Fermentierung ist vorzugsweise ein kontinuierlicher Prozess - um wie einleitend erwähnt den Vollastbetrieb ohne Unterbrechung ausnutzen zu können. Vorzugsweise wird die Fermentierung kontinuierlich ohne Unterbrechung über einen Zeitraum von mindestens 3 Tagen durchgeführt, speziell bevorzugt ist der Zeitraum mindestens 4, 5, 6, 7, 8, 9, 10, 12, 15 ,20, 25, 30, 35, 40, 45, 50, 60 Tage oder mehr, oder jeder Zeitraum innerhalb dieser Tagesanzahlen. Die erfindungsgemäße Anlage hat hierfür vorzugsweise eine Biomasseeinspeisevorrichtung zur kontinuierlichen Einfuhr von Biomasse in den Fermenter. Die Biomasse kann vor der Einspeisung oder im Fermenter auf die nötige Wärme und Feuchtigkeitsgehalte zur Fermentierung vorbereitet werden.

Optional kann vor der Fermentierung die Biomasse, insbesondere mechanisch, zerkleinert werden. Die Zerkleinerung kann bereits den Abbau der Biomasse z.B. durch Verwitterung oder Abbau von Mikroorganismen bedeuten, weswegen sie unmittelbar vor der weiteren Fermentierung vorgenommen werden sollte. Auch dieser Aufschlussprozess ist wie der Fermentationsprozess kein schneller Prozess, sondern als kontinuierlicher Prozess an den Fermentierungsprozess gekoppelt. Der Aufschluss bedingt somit eine hohe Ausbeute an Rohbiogas aus Reststoffen.

Vorzugsweise wird im Reinigungsschritt Methan auf einen Anteil von mindestens 80%, im Speziellen mindestens 85%, mindestens 90 %, mindestens 95%, oder mindestens 97% (alle %-Angaben in vol.-%) aufkonzentriert. Zur Reinigung sind diverse Verfahren bekannt, die eine Gaswäsche, Gaspermeation, Gasadsorption oder Kombinationen davon umfasst. Vorzugsweise kommt eine Gaswäsche zum Einsatz, welche mit einem oder mehr Gaspermeationsschritten kombiniert werden kann (z.B. wie in der Anmeldung EP 12159508.6, oder den Veröffentlichungen DE 10 2005 051 952, WO 2008/034473, DE 10 2008 058 114, US2011/005392, DE 199 47 339, WO 02/26359, US 5,674,629, WO 2008/077837, DE 10 2010 006 649, WO 2006/006164 beschrieben). Alternativ können ein, zwei, drei oder mehr serielle Gaspermeationsschritte zum Einsatz kommen, in denen graduell die Methankonzentration erhöht wird. Vorzugsweise ist die Methankonzentration zwischen 80% und 90% (vol.-%).

Ein Verfahren zur Permeation (Membranseparation) wird beispielsweise in der US 2009/156875 beschrieben und kann erfindungsgemäß eingesetzt werden. Geeignete Membranen zur Trennung von CO₂ und Methan sind im Patent US 5,674,629 beschrieben, z.B. Polyimid-Membranen. Kurz, die Membrantrennung nutzt die unterschiedlichen Membranpermeationseigenschaften unterschiedlicher Gaskomponenten, auch von Methan und CO₂, um diese voneinander zu trennen. Bei Polyimidmembranen ist die Permeationsdifferenz von Methan und CO₂ relativ hoch, wobei CO₂ wesentlich schneller die Membran passiert. Bei gängigen Membranen gilt folgende Reihenfolge des Permeationsverhaltens von schnell zu langsam: H₂O, H₂, He, CO2, O₂, N₂, CH₄. Grundsätzlich ist es möglich, nur mit Membrantrennstufen Produktkonzentrationen von 99% Methan bzw. Kohlendioxid zu erzielen. Mit steigernder Konzentration im Reingas steigt bei diesem Verfahren jedoch der Energieaufwand für die Gasverdichtung und es ist eine mehrstufige Verfahrensführung notwendig.

Alternativ zur Membrantrennung kann auch eine Adsorption, z.B. durch eine PSA, vorgenommen werden. In der PSA wird diskontinuierlich Kohlendioxid (oder alternativ Methan) adsorbiert, wobei ein Kohlendioxid-abgereichertes Produktgas abgegeben wird und infolge Kohlendioxid (oder Methan) wieder desorbiert, wobei ein kohlendioxidreiches Gas - separat vom Kohlendioxidabgreicherten Produktgas - abgegeben wird. Meist werden zwei Adsorber vorgesehen, wobei sich die Adsorption/Desorption zwischen den beiden Adsorbern wechselseitig abwechselt. Adsorber sind beispielsweise in der US 2011/005392 genannt. Die Adsorber können auch durch chemische Absorber ergänzt oder ersetzt werden, welche ebenfalls zur diskontinuierlichen Absorption oder Desorption geeignet sind. Vorzugsweise erfolgt der Wechsel zwischen Absorption und Desorption durch die Anlegung eines unterschiedlichen Druckes. Beispielsweise kann für die Absorption ein hoher Druck (Überdruck) und zur Desorption ein niedriger Druck (Unterdruck, Vakuum) eingestellt werden - z.B. durch Regelung am Produktgasausgang. Alternativ oder zusätzlich kann - je nach Adsorbermaterial - Adsorption und Desorption durch unterschiedliche Temperaturen reguliert werden.

Bei der Gaswäsche wird das Rohbiogas nach der Fermentation gegen eine Waschflüssigkeit geführt (z.B. im Gegenstrom oder im Gleichstrom), wobei die Flüssigkeit CO₂ aufnimmt und Methan als Gas zurücklässt. Die Flüssigkeit kann beispielsweise Wasser sein, welches vorzugsweise unter hohem Druck (z.B. 8-30 bar) Kohlendioxid aufnimmt. Höhere Absorptionen können durch Kohlendioxid-absorbierende Substanzen erzielt werden, die Kohlendioxid chemisch binden. Vorzugsweise enthält die Flüssigkeit eine solche Kohlendioxid-absorbierende Substanz. Kohlendioxid absorbierende Substanzen sind hinlänglich bekannt und sind beispielsweise Carbonat-, Hydrogencarbonat- oder Bicarbonatbilder, wie beispielsweise Kalk, Dolomit, CaO, Ca(OH)₂, Me₂CO₃, wobei Me ein einwertiges Metallion ist (bildet 2 MeHCO₃ mit Wasser und CO₂), Basen oder Amine, insbesondere Alkyl- oder Alkoholamine, wie beispielsweis in der DE 10 2009 056 661 A1 beschrieben. Vorzugsweise ist Me K oder Na. Geeignete Amine sind beispielsweise MEA (Monoethanolamin), DEA (Diethanolamin), TEA (Triethanolamin), Diethyldiamin, 1,4-Diethylendiamin (Piperazin). Vorzugsweise wird ein Karbonat (vorzugsweise K₂CO₃) zusammen mit einem Amin als Impfstoff verwendet. Der Impfstoff wird in geringen Dosen eingesetzt und erhöht katalytisch die Aufnahme von CO₂ durch das Karbonat. Vorzugsweise ist der Impfstoff Piperazin.

Durch die Verwendung eines zweistufigen Absorbers können zumindest 30 vol.-% an CO₂ aus dem Rohbiogas abgetrennt werden. Je nach molaler Mischung an Kaliumcarbonat und Piperazine kann die Absorptionsleistung auf 40% gesteigert werden.

Das durch den Waschprozess gewonnene und im Rahmen der Regeneration abgetrennte Kohlendioxid (CO₂) kann in einer gekoppelten DME-Herstellung, Schritt d), verwertet werden.

Vorzugsweise wird das Rohbiogas vor der Aufreinigung verdichtet, um die Druckverluste während der Aufreinigung zu überwinden.

Als weiterer Reinigungsschritt können Feststoffe wie Si-(insbesondere Siloxane) und S-Partikel und/oder NH₃ entfernt werden. Vorzugsweise wird auch H₂S entfernt. Diese Stoffe können z.B. durch entsprechende Filterung oder Wäscher abgereichert werden. Filter sind beispielsweise Aktivkohlefilter, deren Wirkungsgrad durch den im Rohbiogas befindlichen Wasserdampf erhöht wird.

Vorzugsweise wird nach der Reinigung das Rohbiogas getrocknet, z.B. durch Kühlung und Ausfällung von Wasserkondensat.

In Schritt c) bzw. im Synthesegasreaktor wird Methan des aufgereinigten Biogases mit Sauerstoff und Kohlendioxid zu Synthesegas umgesetzt. Obwohl Methan und Kohlendioxid bereits im Rohbiogas vorhanden sind, wird bevorzugt zunächst eine Trennung vorgenommen, um die Anteile dieser Gase, insbesondere das Methan : Kohlendioxid Molverhältnis genau regulieren zu können. Für eine spezielle Regulation kann auch hochaufgereinigtes Biomethan (z.B. Methangehalt mindestens 95 vol.-%) verwendet werden. Vorzugsweise ist das molare Verhältnis Methan : Kohlendioxid zwischen 3:1 und 1:1, insbesondere bevorzugt zwischen 2,5:1 und 1,5:1, im Speziellen ist es 2:1.

Im Synthesegasreaktor laufen folgende Reaktionen ab: CH₄ + CO₂ → 2 CO + 2 H₂; CH₄ + H₂O → CO + 3 H₂; 2 CH₄ + O₂ → 2 CO + 4 H₂; CO + H₂O ↔ CO₂ + H₂. Durch Regelung des Einlasses von O₂ (und konstantem CH₄ Einlass) und optional des Einlasses von CO₂ kann das Produktverhältnis von CO zu H₂ gesteuert werden, üblicherweise im Bereich zwischen 4:1 und 1:4. Die Verhältnisse können in an sich bekannter Weise gesteuert werden (z.B. WO 2008/138899 A1). Bei Verwendung von Wasserstoff als reduktiv wirkende Spezies entsteht aufgrund der vorliegenden stöchiometrischen Verhältnisse 1 Mol Wasser pro Mol erzeugtem Kohlenmonoxid. Bei Umsetzung von Kohlendioxid mit Methan entstehen 2 Mol Kohlenmonoxid und 2 Mol Wasserstoff, also ein Synthesegas mit dem Kohlenmonoxid zu Wasserstoff-Verhältnis von 1 zu 1 (CO/H₂ gleich 1:1). Bei der Umsetzung von 2 Mol Kohlendioxid mit Ethan entstehen 4 Mol Kohlenmonoxid und 3 Mol Wasserstoff, also ein Synthesegas mit dem Kohlenmonoxid zu Wasserstoff-Verhältnis von 4 zu 3 (CO/H₂ gleich 4:3). Bei der Umsetzung von 3 Mol Kohlendioxid mit 1 Mol Propan entstehen 6 Mol Kohlenmonoxid und 4 Mol Wasserstoff, also ein Synthesegas mit dem Kohlenmonoxid zu Wasserstoffverhältnis von 3 zu 2 (CO/H₂ gleich 3:2). Bei der Umsetzung von 4 Mol Kohlendioxid mit 1 Mol Butan entstehen 8 Mol Kohlenmonoxid und 5 Mol Wasserstoff, also ein Synthesegas mit dem Kohlenmonoxid zu Wasserstoff-Verhältnis von 8 zu 5 (CO/H2 gleich 8:5). Dies sind nur Beispiele für Kontrollmöglichkeiten. Vorzugsweise hat das Synthesegas, welches in Schritt c) erhalten wird und/oder in Schritt d) eingespeist wird, ein molares Verhältnis von Kohlenmonoxid : Wasserstoff von zwischen 1:2 bis 2:1, vorzugsweise von 1:1,5 bis 1,5:1. Insbesondere bevorzugt ist das Verhältnis 1:1, da damit in Schritt d) die DME-Ausbeute erhöht werden kann (im Vergleich zur Produktion von Methan als Nebenprodukt).

Vorzugsweise erfolgt die Reaktion des Schrittes c) bei einer Temperatur zwischen 550°C und 1000°C, vorzugsweise zwischen 630°C und 900°C, insbesondere bevorzugt zwischen 750°C und 850°C. Die nötige Wärme kann durch Wärmetauscher und/oder die Verbrennung von (Roh-)Biogas erhalten werden. Der Druck in Schritt c) ist vorzugsweise zwischen 20 bar und 80 bar, insbesondere zwischen 22 bar und 60 bar, im Speziellen zwischen 25 bar und 40 bar.

Die Synthesegasreaktion kann trocken oder nass erfolgen. Zur nassen Reaktion kann Wasser, insbesondere Wasserdampf eingeleitet werden.

Als weitere vorzugsweise Regelgröße wird der eingeleitete Sauerstoff verwendet. Dadurch kann das molare Verhältnis von Kohlenmonoxid : Wasserstoff im erhaltenen Synthesegas durch Regelung des Sauerstoffeinlasses gesteuert werden. Hierbei werden die Produktgasverhältnisse gemessen und je nach Verteilung kann durch Modifizierung des Sauerstoffeinlasses die Verteilung hin zum gewünschten Verhältnis verschoben werden.

Auch für die Erzeugung der notwendigen Wärme im Synthesegasreaktor, jene Wärme, die notwendig ist, um den Umwandlungsprozess der Ausgangsstoffströme zu dem gewünschten synthetischen Gasgemisch aus Kohlendioxid (CO) und Wasserstoff (H2) im molaren Verhältnis von ungefähr 1:1 zu erreichen kann, als Regelgröße flüssiger Sauerstoff verwendet werden. Dieser wird entspannt, im Zuge der Entspannung verdampft und dann verdichtet und so dem Synthesegasprozess (autotherme Reaktion) zugeführt. Zusätzlich werden vorzugsweise Wasserdampf und Kohlendioxid der Reaktion zugeführt. Kohlendioxid kann aus der Biomethanreinigung zur Verfügung gestellt werden.

Als Bauform für den Synthesegasreaktor wird vorzugsweise das Gegenstromprinzip verwendet, das das Reaktionsgas am Reaktorboden einleitet, und über interne Rohrführungen erwärmt, sodass ein Teil der Reaktionswärme zum Aufheizen des Reaktionsgases verwendet wird. Der Rest der überschüssigen Wärme kann durch Kühlelemente entnommen werden.

Vorzugsweise wird Schritt c) bei einem Druck von mindestens 20 bar (2 MPa) durchgeführt. Hierfür kann das Rohbiogas verdichtet werden und/oder verdichtetes Kohlendioxid zur Druckerhöhung verwendet werden. Als Kohlendioxidquelle kann in Schritt b) abgetrenntes Kohlendioxid zum Einsatz kommen.

Die Produktion eines Methanol/DME-Gemisches aus Synthesegas ist an sich bekannt, wie einleitend beschrieben wurde. Erfindungsgemäß ist Methanol ein unerwünschtes Nebenprodukt. Daher wird durch Steuerung der Reaktionsbedingungen und der Verhältnisse der Ausgangsstoffe die Ausbeute von DME optimiert. Hierzu sollte wie bereits erwähnt das molare Verhältnis von Kohlenmonoxid : Wasserstoff im Synthesegas zwischen 1:2 und 2:1 sein, vorzugsweise ist es zwischen 1:1,5 und 1,5:1, insbesondere 1:1. Vorzugsweise erfolgt die Reaktion des Schrittes d) bei einer Temperatur zwischen 200°C und 320°C, vorzugsweise zwischen 220°C und 300°C, insbesondere bevorzugt um 250°C. Hierzu wird das nach Schritt c) erhaltene heißere Synthesegas abgekühlt, vorzugsweise über einen Wärmetauscher, wobei andere Ausgangsgase, z.B. das aufgereinigte Rohbiogas für den Synthesegasreaktor, Schritt c)=, erwärmt werden können. Der Druck in Schritt d) ist vorzugsweise zwischen 20 bar und 80 bar, insbesondere zwischen 22 bar und 60 bar, im Speziellen zwischen 25 bar und 40 bar.

Im DME-Reaktor, Schritt d), verbindet sich Kohlendioxid und/oder Kohlenmonoxid jeweils mit Wasserstoff zu Methanol, unter Abspaltung von Wasser und die anschließende Kondensation von Methanol zu DME und Abspaltung von Wasser. Dies kann unter der Bildung von Methanol als Zwischenstufe erfolgen (Zweischrittprozess) oder unter direkter Umsetzung (Einschrittprozess). Welche Schrittfolge abläuft hängt vom eingesetzten Katalysator ab. Vorzugsweise werden Katalysatoren für den Einschrittprozess verwendet, z.B. Cu/ZnO/Al₂O₃-Katalysatoren. Der DME-Reaktor enthält vorzugsweise eine Schüttung des Katalysators. Dennoch können gewisse Mengen Methanol anfallen, welches vorzugsweise vom heißen Produktgas ausgefällt wird (z.B. bei etwa 150°C und 30 bar) und dem Prozess, Schritt d), wieder zurückgeführt wird. Andere Prozessgase, wie CO, CO₂ und H₂, können nach Ausfällung von DME (z.B. zwischen 50°C und 30 bar und 25°C und 3 bar) dem Synthesegasreaktor, Schritt c), zugeführt werden.

Vorzugsweise wird Schritt d) bei einem Druck von mindestens 20 bar (2 MPa) durchgeführt. Hierfür kann das Synthesegas verdichtet werden und/oder verdichtetes Kohlendioxid zur Druckerhöhung verwendet werden. Als Kohlendioxidquelle kann in Schritt b) abgetrenntes Kohlendioxid zum Einsatz kommen.

Nicht reagiertes Abgas aus Schritt d) kann vorzugsweise der Synthesegasproduktion nach Schritt c) zugeführt wird.

DME kann, wie in US 5,0375,511 beschrieben, destilliert bzw. aufkonzentriert und gereinigt werden.

Die Erfindung betrifft auch eine Produktionsanlage, geeignet zur Herstellung von Dimethylether mit einem Fermenter, einem Rohbiogasreiniger zur Trennung von Methan und Kohlendioxid, mit einem Synthesegasreaktor und einem Dimethylether-Reaktor zur katalytischen Kondensation von Synthesegas zu Dimethylether. Diese Bauteile bzw. die Reaktionsschritte darin wurden oben bereits ausführlich diskutiert. Zum Transport zwischen den Anlagenteilen sind diese in der gegebenen Reihenfolge vorzugsweise mit Leitungen verbunden.

Vorzugsweise enthält die Produktionsanlage weiter einen Druckspeicher zur Lagerung von Methan und einem weiteren Druckspeicher zur Lagerung von unter Druck verflüssigtem Dimethylether. Zusätzlich enthält die Anlage weiter Zapfsäulen zur Betankung von Kraftfahrzeugen mit Methan und/oder Dimethylether.

Die Kombination der Verwertung von Rohbiogas oder von Biomethan in Biomethan-Tankstellen und der Verwertung von Bio-Dimethylether (Bio-DME) stellt eine flexible in sich geschlossene Verfahrenskombination dar, die von der zufälligen Nutzung des Biomethans im Rahmen eines Tankprozesses in der Biomethan-Tankstelle unabhängig macht, und so den Erzeugungsprozess von Rohbiogas in Form eines Konstantlastprozesses umsetzen lässt.

Auch Methan kann zur Betankung verwendet werden. Das gewonnene und aufkonzentrierte Biogas (z.B. Methan-Anteil mindestens 95 vol.-%) kann für eine Biomethan-Tankstelle genutzt werden. Die Biomethan-Tankstelle besteht vorzugsweise aus einem Hochdruckverdichter, der das Biomethan in einen kleinen Gasbündelspeicher für den Kurzzeitbetrieb an der Zapfsäule speichert. Um den Tankprozess mit dem Fermentierungsprozess zu koppeln, besteht die Notwendigkeit, den konstanten Rohbiogasproduktionsprozess aufrecht zu erhalten, und daher das anfallende restliche in der Biomethan-Tankstelle nicht verbrauchte Biogas und/oder Biomethan einer weiteren Verwertung zuzuführen. Dies wird durch die Produktion von DME bewerkstelligt.

Weiters betrifft die Erfindung die Verwendung einer Produktionsanlage zur Herstellung von Dimethylether bzw. ebenso die Verwendung der Anlage zur Betankung von Kraftfahrzeugen mit DME und/oder Methan.

Die vorliegende Erfindung wird durch die folgenden Figuren und Beispiele näher beschrieben, ohne auf diese konkreten Ausführungsformen der vorliegenden Erfindung limitiert zu sein.

Gezeigt ist in den Figuren schematisch:
Fig. 1 eine Anlage zur Aufbereitung von Rohbiogas, das für den weiteren Verwertungsprozess benötigt wird;
Fig. 2 eine Anlage mit einem zweistufigen Waschsystem zur Aufbereitung zur Aufkonzentration des Rohbiogases im Methangehalt, indem das im Rohbiogas enthaltene CO₂ teilweise aus dem Rohbiogas mit einer Waschflüssigkeit absorbiert wird;
Fig. 3 eine Anlage zur Aufbereitung des Rohbiogases und die Aufsplittung in einzelne Stoffströme, bedingt durch die Aufkonzentration durch das Waschsystem, und die Aufkonzentration zu Biomethan;
Fig. 4 eine Anlage zur Koppelung der direkten DME-Synthese auf der Basis von Rohbiogas mit dem Biogasaufbereitungsprozesses;
Fig. 5 eine Anlage mit der Koppelung der Biogasaufbereitung mittels zweistufiger Gaspermeation; und
Fig. 6 eine Anlage mit der Koppelung der Biogasaufbereitung mittels dreistufiger Gaspermeation.

In den Figuren sind identische bzw. einander entsprechende Bereiche, Bauteile, Bauteilgruppen oder Verfahrensschritte mit denselben Bezugszeichen gekennzeichnet. Die Flussrichtungen in Leitungen sind mit Pfeilen gekennzeichnet.

### BEZUGSZEICHENLISTE

### FIGUR 1:

1 Rohbiogas
2 Gas Booster (Niederdruckverdichter)
3 Aktivkohlefilter
4 Absorptionskolonne
5 Absorptionskolonne
6 Regeneriertes Waschfluid für die Absorberkolonne
7 Beladenes Waschfluid aus der Absorberkolonne
8 Trockner
9 Recuperator
10 Kaltwasser
11 Rohbiogas für DME-Prozess
12 Rohbiogas für Biomethanprozess

### Figur 2:

71 Absorberkolonne
72 Waschfluidpumpe
73 Recuperator Wärmetauscher
74 Desorberkolonne
75 Heizwärmetauscher
76 Waschfluidpumpe
77 Antriebsmotor
14 Kohlendioxid aus Regenerationsprozess
1 Rohbiogas Eintritt in Absorber
12 aufkonzentriertes Rohbiogas aus dem Absorber

### Figur 3:

1 Rohbiogas
13 Wärmezuführung Waschprozess
14 Kohlendioxid aus Waschprozess
15 Aufkonzentriertes Rohbiogas
16 Armatur Rohbiogas
17 Armatur Rohbiogas
18 Verdichter vor Biomethanprozess
19 Armatur Rohbiogas
20 Armatur Rohbiogas
21 Permeat Recycle
22 Armatur Permeat Recycle
23 Verdichtetes Rohbiogas
24 Permeat (Kohlendioxid) aus Biomethanprozess
25 Recycle aus Biomethanprozess
26 Armatur Recycle
27 Biomethan
28 Flüssiges Bio-DME
29 Bio-DME Tank
30 Sauerstofftank
31 Verdichter Sauerstoff
32 Sauerstoff für Bio-DME Prozess
33 Armatur Biomethan
34 Armatur Biomethan
35 Biomethan zur Tankstelle
36 Verdichter Biomethan-Tankstelle
37 Biomethan Speicherbündel
38 Biomethan Zapfsäule

### Figur 4:

29 Bio-DME Tank (Flüssiges DME)
30 Flüssiger Sauerstofftank
31 Sauerstoffverdichter
32 Sauerstoffleitung
39 Methanverdichter
40 Verdichtete Methanleitung
41 Verdichter Kohlendioxid
42 Verdichtetes Kohledioxid Leitung
43 Wassertank (Demin)
44 Pumpe für Demin-Wasser
45 Wasserleitung
46 Verdampfer Wasserdampf
47 Pumpe für Kondensat auf Trockner 49
48 Wasserdampf in Synthesegasreaktor
49 Trockner
50 Methanolpumpe
51 Offgas-Verdichter
52 Wärmetauscher Methanolkondensat
53 Wärmetauscher DME Kondensat
54 Verbrennungsluft
55 Rohbiogasbrenner
56 Wärmetauscher im Synthesegasreaktor
57 Abgas aus Gasbrenner

### Figur 5:

17 Rohbiogas
18 Verdichter Feedseite
21 Permeat aus erster Gasmodulstufe 59
26 Permeat Kohlendioxid mit Methanschlupf 35 Biomethan
58 Rententat aus der dritten Gasmodulstufe
59 Erster Gaspermeationsmodul
60 Rententat erste Stufe
61 Zweiter Gaspermeationsmodul
62 Permeat zweite Gaspermeationsmodul
63 Dritter Gaspermeationsmodul

### Figur 6:

17 Rohbiogas
21 Permeat aus zweiter Gaspermeationsstufe = Recycle
18 Verdichter
26 Kohlendioxid mit Methanschlupf erste Gaspermeationsstufe
35 Biomethan Rententat aus zweiter Gaspermeationsstufe
59 Erste Gaspermeationsstufe
60 Rententat erste Gaspermeationsstufe
61 Zweite Gaspermeationsstufe

### Zusammenfassung der Prozessgruppen:

100 Rohbiogasaufbereitung (inkl. Waschprozess)
101 Aufreinigung zur Verwertung von Biomethan
102 DME-Prozess
103 Biomethan-Tankstelle
104 Synthesegasreaktor
105 DME-Reaktor

### BEISPIELE

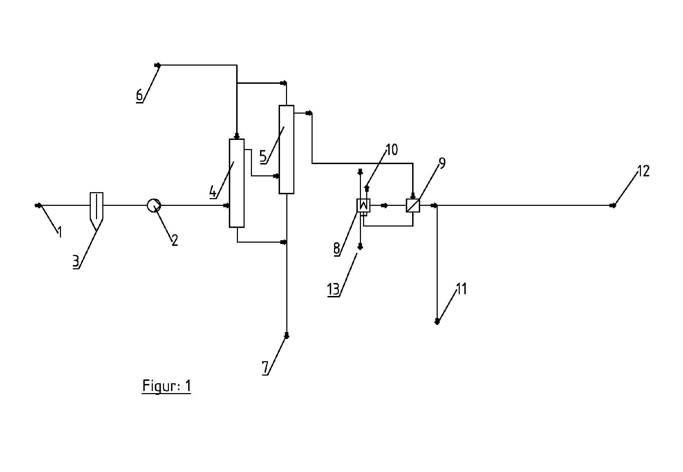

Fig. 1 zeigt das erfindungsgemäße Verfahren zur Reinigung von Rohbiogas bestehend aus dem Rohbiogas, das über den Rohbiogas Booster angesaugt wird, und über den Aktivkohlefilter 3 zur Reduktion des Schwefelwasserstoffes, dann dem zweistufigen Absorber 4,5 zugeführt wird, bestehend aus dem Absorber 4 und dem nachgeschalteten Absorber 5. In der Folge wird das so upgegradete Rohbiogas dem Trockner 8,9 zugeführt. Der Trockner 8,9 besteht aus zwei miteinander gekoppelten Wärmetauschern 8,9, die aus einem Trockner 8 und einem Rekuperator 9 bestehen. Der Trockner wird mit Kaltwasser 10 2°C bis 5°C Vorlauftemperatur betrieben und besitzt die Option demineralisiertes Wasser in den Trockner eindüsen zu können, um so den Ammoniakgehalt weiter senken zu können. Ammoniak hat die Eigenschaft, wasserlöslich zu sein. Da das Wasser zerstäubt wird und als Nebel in Form von feinen Tröpfchen im Rohbiogasstrom eingedüst wird, hat der mit dem Rohbiogas mitgeführte Ammoniak die Eigenschaft sich an die Wassertröpfchen zu binden, und über den Kondensatablass 13 aus dem Trockner zu schleusen. Nach dem Trockner wird das Rohbiogas wieder aufgewärmt und der weiteren Verwertung zugeführt. Wie in der Figur 1 gezeigt, kann nach dem Trocknungsprozess das aufbereitete und aufkonzentrierte Rohbiogas 11 der Verwertung im DME-Prozess oder der Verwertung im Gaspermeationsprozess zugeführt werden. Alternativ kann das so aufbereitete Rohbiogas 12 dem Prozess der Biomethanerzeugung und der Verwertung in CNG-Tankstellen zugeführt werden.

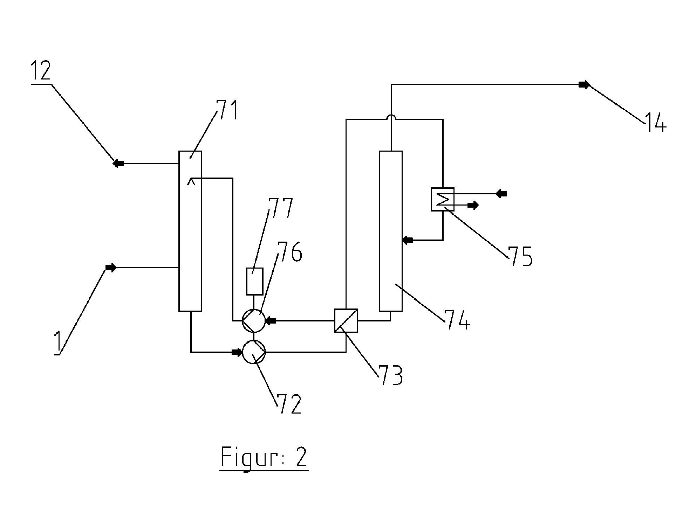

Fig. 2 zeigt ein erfindungsgemäßes Verfahren des Biogas-Upgradings bestehend aus einer Absorptionskolonne 71 und einer Desorptionskolonne 74. Unter Biogas Upgrading versteht man das Herauswaschen eines Anteiles an Kohlendioxid, um so den Methangehalt im verbleibenden Rohbiogas zur erhöhen. Das mit Kohlendioxid (CO₂) beladene Waschfluid, eine Mischung aus Wasser mit Kaliumkarbonat und Piperazine, wird über die Pumpe 72 und dem Recuperator Wärmetauscher 73 der Desorberkolonne 74 zugeführt. Das regenerierte Waschfluid wird von der Pumpe 76 angesaugt und der Absorptionskolonne 71 zugeführt. Im Desorber 74 wird das Kohlendioxid im dampfförmigen Zustand 14 zur weiteren Verwertung für den DME Prozess abgeleitet. Das in der Methankonzentration upgegradete Rohbiogas 12 wird dem Trocknungsprozess und dem Aufbereitungsprozess für Biomethan oder dem DME-Prozess zugeführt.

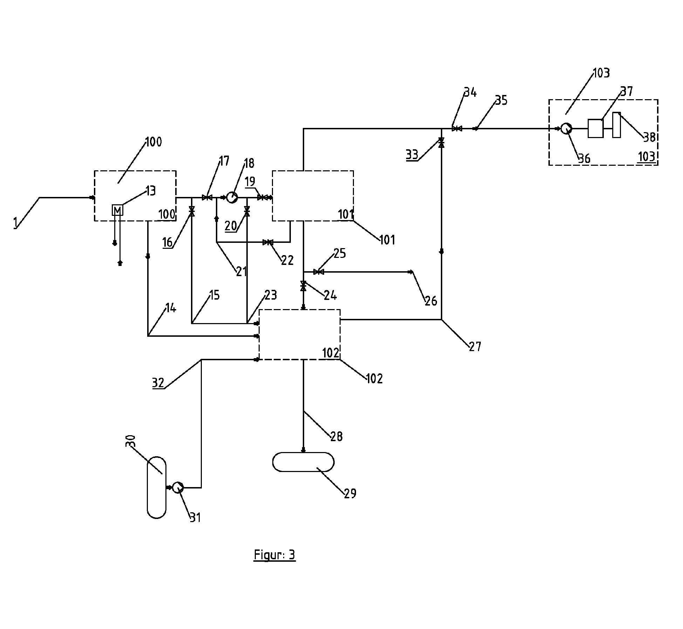

Fig. 3 zeigt eine erfindungsgemäße Kombination vom Biogas-Aufkonzentrationsprozess 100, dem Biomethan-Prozess 101, dem Bio-DME-Prozess 102, und der Nutzung von Biomethan in Form einer Biomethan-Tankstelle 103. Das Rohbiogas 1 aus dem Fermentierungsprozess wird nach der Rohbiogasaufbereitung dem Aufkonzentrationsprozess 100 zugeführt, in dem ein Teil des im Rohbiogas vorhandenen Kohlendioxids (CO₂) herausgewaschen wird und in der Folge im Rahmen der Regeneration des Waschfluides aus dem Waschfluid wieder in dampfförmigem Zustand gewonnen wird. Das so gewonnene Kohlendioxid (CO₂) 14 wird dem DME-Prozess 102 zugeführt. Je nach Nutzung des Rohbiogases kann dieses nach dem Trocknungsprozess mit schwachem Druck 15 dem DME-Prozess zugeführt werden, oder man nutzt die Verdichtung mittels Kompressor 18 aus, und führt das verdichtete Rohbiogas 23 dem DME-Prozess zu. Neben dem Kohlendioxid 14 und dem Rohbiogas 15 oder 23 wird dem DME-Prozess auch Sauerstoff aus einem Flüssigtank 30 mit Verdampfung und anschließender Verdichtung 31 über die Leitung 32 zugeführt. Das aus dem DME-Prozess 103 gewonnene flüssige DME 28 wird in einem Tank 29 gelagert und gespeichert. Für den DME-Prozess kann aber auch Biomethan 27, das aus dem Biomethanprozess 101 gewonnen wird, verwertet werden. Der Biomethanprozess 101 arbeitet nach dem Prinzip der Gaspermeation, wobei das Biomethan 35 entweder der Biomethan-Tankstelle zugeführt werden kann, oder wenn die Speicher 37 der Tankstelle voll sind und kein Bedarf besteht, über Biomethanleitung 27 dem DME-Prozess zugeführt werden kann. Das aus dem Biomethanprozess gewonnene Permeat 26 wird entweder der Verflüssigung oder thermischen Oxidation oder der zusätzlichen Verwertung 24 im DME-Prozess zugeführt. Die Figur 3 zeigt somit die Verschränkung und Kombination der Erzeugung von Biomethan 101 mit der Biomethan-Tankstelle 103 und dem DME-Prozess, in dem das Rohbiogas 15 oder 23 und/oder das Biomethan 27 zu Bio-DME verwertet wird, das in flüssiger Form im Tank 29 gespeichert werden kann.

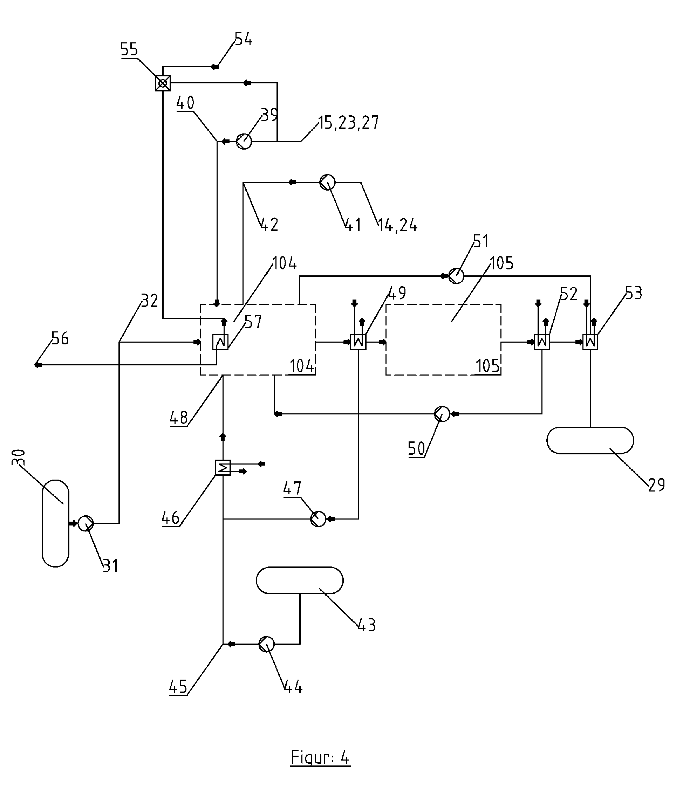

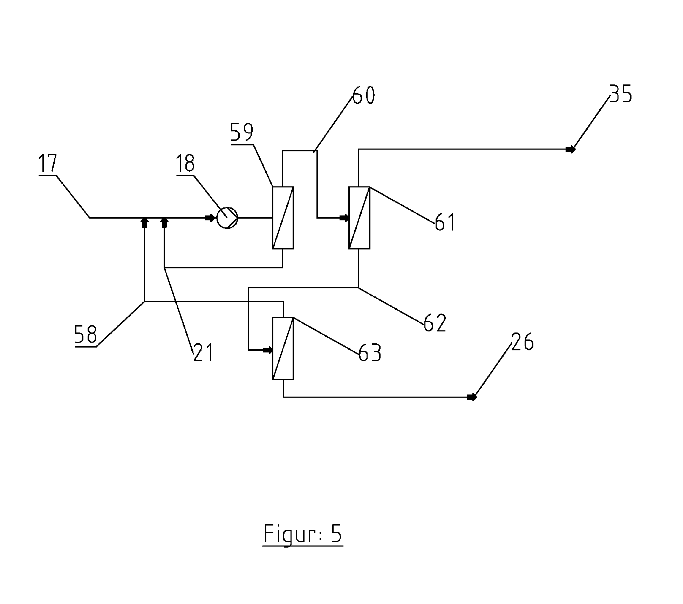

Fig. 4 zeigt ein erfindungsgemäßes Vorfahren zur Erzeugung von Bio-DME in dem Rohbiogas 15 oder 23 und/oder Biomethan 27 zugeführt werden, und über den Verdichter 39 als verdichtetes Methan 40 dem Synthesegasreaktor 104 zugeführt wird. Der Synthesegasreaktor 104 hat die Aufgabe, ein synthetisches Gasgemisch bestehend aus Kohlenmonoxid (CO) und Wasserstoff (H₂) in einem molaren Verhältnis 1:1 zu erzeugen und der Trocknung 49 zuzuführen. Neben dem Methan wird Kohlendioxid benötigt, das über die aus dem Waschprozess 100 und/oder dem Biomethanprozess 24 dem Verdichter 41 zugeführt wird und in verdichteter Form 42 dem Synthesegasreaktor 104 zugeführt wird. Für den Synthesegasreaktor wird Sauerstoff benötigt, der als flüssiger Sauerstoff 30 nach einer Entspannung und Verdichtung 31 dem Synthesegasreaktor zugeführt wird. Für die Regelung der chemischen Prozesse im Synthesegasreaktor wird Wasserdampf benötigt, der mit Wasser aus dem Tank 43 über eine Pumpe 44 auf den notwendigen Betriebsdruck des Synthesegasreaktors 104, einem Verdampfer 46 zugeführt wird. Das aus der Trocknung 49 anfallende Wasser wird über eine Pumpe 47 verdichtet und dem Verdampfer 46 zugeführt. Das aus dem Synthesegasreaktor anfallende Gasgemisch wird nach der Trocknung 49 dem DME-Reaktor zugeführt, in dem mittels eines Katalysators die Bildung von DME angeregt wird. Neben DME entstehen geringe Mengen Methanol, das über den Wärmetauscher 52 als Kondensat abgeschieden werden. Bei der weiteren Abkühlung über den Wärmetauscher 53 wird DME in flüssiger Form abgeschieden und das Offgas, bestehend aus den Resten von Kohlenmonoxid (CO), Wasserstoff (H₂) und Kohlendioxid (CO₂) und nicht verbrauchtem Sauerstoff (O₂) wird einem Verdichter 51 zugeführt und danach in den Synthesegasreaktor 104 miteingetragen. Um den Reaktor aus dem kalten Zustand anzufahren und auf eine Starttemperatur von 600°C zu bringen, wird Rohbiogas 14 verwendet. Hier stellt sich der niedrige Druck des Rohbiogases nach dem Waschprozess 100 als Vorteil dar, da für die Verbrennung in einer Brennkammer 55 zur Regelung zusammen mit der notwendigen Verbrennungsluft 54 die notwendige Wärme 56 über das Abgas 57 dem Synthesegasreaktor 104 zur Verfügung gestellt wird. Fig. 5 zeigt eine erfindungsgemäße Vorrichtung bestehend aus einem dreistufigen Aufbereitungsprozess zur Erzeugung von Biomethan mittels dem Gaspermeationsverfahren. Der dreistufige Prozess besteht aus drei Gasmembranmodulen, wobei das Rohbiogas 17 mit dem Permeat 62 aus der zweiten Membranstufe 61 und dem Rententat 58 aus der dritten Membranstufe 63 gemischt wird und dem Verdichter 18 zugeführt wird. Das verdichtete Feedgas wird der ersten Stufe Gasmembran 59 zugeführt. Das Permeat 21 der ersten Stufe 59 wird dem Rohbiogas 17 zugeführt. Das Rententat 60 aus der ersten Stufe Gasmembran 59 wird der zweiten Stufe Gasmembranmodul 60 zugeführt. Das Rententat der zweiten Stufe Gasmembranmodul 35 wird als Biomethan der Biomethantankstelle 103 oder der Verwertung im DME-Prozess 102 über die Leitung 27 zugeführt. Das Permeat 26 aus der dritten Stufe Gasmembranmodul 63 wird einer Verwertung im DME-Prozess 102 zugeführt oder mit einem thermischen Oxidator zu Restwärme umgewandelt, und so mögliche flüchtige Kohlewasserstoffe verwertet.

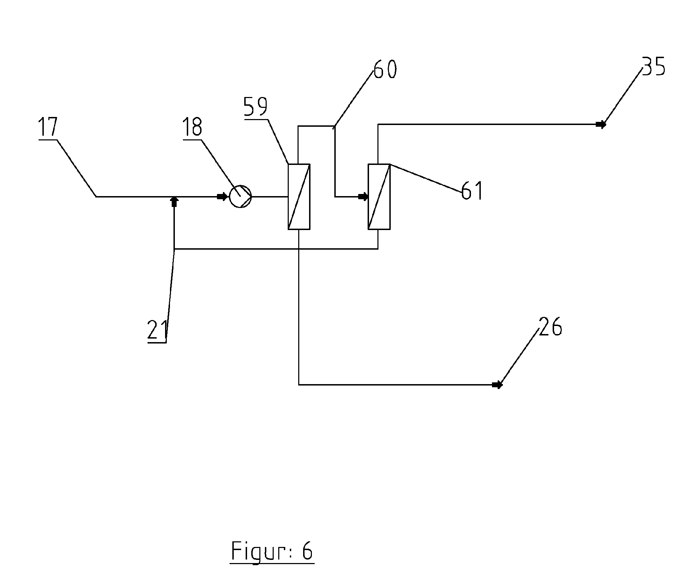

Fig. 6 zeigt eine erfindungsgemäße Vorrichtung bestehend aus einem zweistufigen Aufbereitungsprozess zur Erzeugung von Biomethan mittels dem Gaspermeationsverfahren. Der zweistufige Prozess besteht aus zwei Gasmembranmodulen, wobei das Rohbiogas 17 mit dem Permeat 62 aus der zweiten Membranstufe 61 gemischt wird und dem Verdichter 18 zugeführt wird. Das verdichtete Feedgas wird der ersten Stufe Gasmembran 59 zugeführt. Das Permeat 21 der ersten Stufe 59 wird dem Rohbiogas 17 zugeführt. Das Rententat 60 aus der ersten Stufe Gasmembran 59 wird der zweiten Stufe Gasmembranmodul 60 zugeführt. Das Rententat der zweiten Stufe Gasmembranmodul 35 wird als Biomethan der Biomethan-Tankstelle 103 oder der Verwertung im DME-Prozess 102 über die Leitung 27 zugeführt. Das Permeat 26 aus der ersten Stufe Gasmembranmodul 59 wird einer Verwertung im DME Prozess 102 zugeführt oder mit einem thermischen Oxidator zu Restwärme umgewandelt, und so mögliche flüchtige Kohlenwasserstoffe verwertet.

## Patentansprüche

1. Verfahren zur Herstellung von Dimethylether, umfassend die Schritte der
a) Fermentierung von Biomasse zur Erzeugung von Rohbiogas enthaltend Methan und Kohlendioxid,
b) Reinigung des Rohbiogases durch Abtrennung von Kohlendioxid, wodurch ein Methan-angereichertes Rohbiogas erhalten wird,
c) Herstellung von Synthesegas umfassend Kohlenmonoxid und Wasserstoff durch Umsetzung von Methan mit Sauerstoff,
d) Herstellung von Dimethylether aus dem Synthesegas durch katalytische Kondensation des Synthesegases oder von dem Synthesegas als Zwischenschritt kondensiertem Methanol.

2. Verfahren nach Anspruch 1, **dadurch gekennzeichnet, dass** durch die Fermentierung der Biomasse ein Rohbiogas umfassend 40% bis 65% (vol.-%) Methan und 35% bis 55% (vol.-%) Kohlendioxid erhalten wird.

3. Verfahren nach Anspruch 1 oder 2, **dadurch gekennzeichnet, dass** die Fermentierung den mikrobiellen Abbau organischer Stoffe unter anoxischen Bedingungen umfasst.

4. Verfahren nach einem der Ansprüche 1 bis 3, **dadurch gekennzeichnet, dass** die Fermentierung Abbau von organischen Stoffen ausgewählt aus Pflanzensilage, insbesondere Maissilage oder Grassilage, Hirse, Stroh, insbesondere Maisstroh oder Getreidestroh, Gras, Klee, Luzerne, Viehmist oder -dung, insbesondere Schweine-, Hühner-, Kuh-, oder Pferdemist oder Gülle, Bioabfall oder -müll, Haushalts-Biomüll, Pflanzenschlempe, Maische oder Kompost, Industrieabfall der Getränkeindustrie, Industrieabfall der Lebensmittelindustrie, Brotreste, verdorbene Lebensmittel, umfasst.

5. Verfahren nach einem der Ansprüche 1 bis 4, **dadurch gekennzeichnet, dass** im Reinigungsschritt Methan auf einen Anteil von mindestens 80% (vol.-%) aufkonzentriert wird.

6. Verfahren nach einem der Ansprüche 1 bis 5, **dadurch gekennzeichnet, dass** der Reinigungsschritt eine Gaswäsche, Gaspermeation, Gasadsorption oder Kombinationen davon umfasst, vorzugsweise eine Gaswäsche.

7. Verfahren nach einem der Ansprüche 1 bis 6, **dadurch gekennzeichnet, dass** im Reinigungsschritt Feststoffe ausgewählt aus Si- und S-Partikeln und/oder NH₃ entfernt werden.

8. Verfahren nach einem der Ansprüche 1 bis 7, **dadurch gekennzeichnet, dass** Schritt c) bei einem Druck von mindestens 20 bar durchgeführt wird, wobei vorzugsweise hierfür das Rohbiogas verdichtet wird und/oder verdichtetes Kohlendioxid zur Druckerhöhung verwendet wird, insbesondere wobei in Schritt b) abgetrenntes Kohlendioxid zum Einsatz kommt.

9. Verfahren nach einem der Ansprüche 1 bis 8, **dadurch gekennzeichnet, dass** das Synthesegas ein molares Verhältnis von Kohlenmonoxid : Wasserstoff von zwischen 1:2 bis 2:1 hat, vorzugsweise von 1:1,5 bis 1,5:1.

10. Verfahren nach einem der Ansprüche 1 bis 9, **dadurch gekennzeichnet, dass** das molare Verhältnis von Kohlenmonoxid : Wasserstoff im Synthesegas durch Regelung des Sauerstoffeinlasses gesteuert wird.

11. Verfahren nach einem der Ansprüche 1 bis 10, **dadurch gekennzeichnet, dass** nicht-reagiertes Abgas aus Schritt d) der Synthesegasproduktion nach Schritt c) zugeführt wird.

12. Verfahren nach einem der Ansprüche 1 bis 10, **dadurch gekennzeichnet**, Schritt d) bei einem Druck von mindestens 20 bar durchgeführt wird, wobei vorzugsweise hierfür das Synthesegas verdichtet wird und/oder verdichtetes Kohlendioxid zur Druckerhöhung verwendet wird, insbesondere wobei in Schritt b) abgetrenntes Kohlendioxid zum Einsatz kommt.

13. Produktionsanlage geeignet zur Herstellung von Dimethylether mit einem Fermenter, einem Rohbiogasreiniger zur Trennung von Methan und Kohlendioxid, mit einem Synthesegasreaktor und einem Dimethylether-Reaktor zur katalytischen Kondensation von Synthesegas zu Dimethylether.

14. Produktionsanlage nach Anspruch 13, weiter mit einem Druckspeicher zur Lagerung von Methan und einem weiteren Druckspeicher zur Lagerung von unter Druck verflüssigtem Dimethylether, wobei vorzugsweise die Anlage weiter Zapfsäulen zur Betankung von Kraftfahrzeugen mit Methan und/oder Dimethylether aufweist.

15. Verwendung einer Produktionsanlage nach Anspruch 13 oder 14 zur Herstellung von Dimethylether nach einem der Ansprüche 1 bis 12.
